# EUROPEAN PATENT APPLICATION

(11) **EP 3 187 103 A1**
(43) Date of publication of application: **05.07.2017**
(21) Application number: 16206947.0
(22) Date of filing: 27.12.2016
(51) Int. Cl.: A61B 5/00, A61B 5/145, A61B 5/1486

(54) **SENSOR SUBSTRATE, ANALYSIS ELEMENT, GLUCOSE MEASUREMENT DEVICE, AND INSULIN SUPPLY DEVICE**

(30) Priority: 28.12.2015 JP 2015256064
(71) Applicant: Seiko Epson Corporation, Tokyo 160-8801 (JP)
(72) Inventor: Fujita, Tetsuji, Suwa-shi, Nagano 392-8502 (JP); Ito, Masaki, Suwa-shi, Nagano 392-8502 (JP)
(74) Representative: Hoffmann Eitle

(57) **Abstract**

A detection section (a sensor substrate according to the invention) is used in such a manner that it is inserted into a living body by being guided by a needle section (insertion needle) to be stuck and inserted into the living body. The detection section includes a first region which is provided in a tip end portion of the detection section and includes an electrode layer (detection electrode), a third region which includes a wiring section and has a smaller width than the width of a slit of the needle section (insertion needle), and a second region which is provided between the first region and the third region and has the same width as the width of the third region by gradually decreasing from the width of the first region.

## Description

### BACKGROUND

### 1. Technical Field

The present invention relates to a sensor substrate, an analysis element, a glucose measurement device, and an insulin supply device.

### 2. Related Art

With the recent increase in health consciousness, health care is performed by measuring a blood glucose level, a lactic acid level, an antibody level, an enzyme level, or the like in a body fluid such as blood, interstitial fluid, saliva, or sweat of a specific individual over time.

For example, for diabetic patients, it is particularly important to measure (monitor) a blood glucose level over time.

Diabetic patients are divided into type I and type II according to the symptoms, however, in both types, insulin secretion from the pancreas is not normal. Due to this, the internal organs cannot normally incorporate glucose, and therefore, metabolic abnormality occurs and also the body weight decreases. Moreover, it is known that when a state where the blood glucose level is high is maintained over a long period of time, a serious complication such as "diabetic retinopathy", "diabetic nephropathy", "diabetic microvascular disease", or "diabetic neuropathy" is developed. The current situation is that in order to prevent the development of such a serious complication, a treatment method in which the blood glucose level is maintained within a normal range by administering insulin by injection is adopted for patients in whom a state where the blood glucose level is high persists.

In type I diabetic patients, insulin is not secreted at all due to a pancreatic disease, and therefore, it is necessary to measure a blood glucose level by blood collection several times a day (at least four times, before meal and before going to bed), and administer insulin. As for the timing of administration, in order to suppress an excess increase in the blood glucose level after meal, the blood glucose level before meal is measured and the calories of the meal is calculated beforehand, and then the dose of insulin is determined and insulin is administered by injection before meal. Further, a symptom called "dawn phenomenon" is known as an increase in the blood glucose level, and the blood glucose level increases at dawn at 8 to 10 hours after going to bed. However, in order to cope with this physiological phenomenon, it is necessary to wake up once before dawn and measure the blood glucose level by blood collection, and if the blood glucose level is high, it is necessary to administer insulin. In this manner, although general healthy individuals can go about daily life without caring about the blood glucose level, however, diabetic patients (particularly type I diabetic patients) are compelled to live a life while caring about the blood glucose level all day long.

The current situation is that in order to reduce the burden of daily life on such patients and families who support the patients, that is, in order to improve the QOL (Quality of Life) of the patients and families of the patients, the development of an artificial pancreas or a device equivalent thereto has been demanded. In view of this, first, it is necessary to measure and control the blood glucose level over time (continuously) and automatically.

For example, a device so-called a continuous glucose monitoring (CGM) device utilizing an enzymatic reaction for use in monitoring a glucose level in the interstitial fluid of a patient over a long period of time by embedding a blood glucose level sensor in a living body (subcutaneous tissue) has been proposed.

The basic principle of the quantitative determination of glucose using an enzymatic reaction is that in the presence of an enzyme (for example, glucose oxidase), when glucose and oxygen are present in the vicinity of the enzyme, gluconic acid and hydrogen peroxide are produced. By measuring the amount of an electric current generated by electrically decomposing the produced hydrogen peroxide, the amount of hydrogen peroxide can be quantitatively determined. Therefore, a glucose level can be calculated based on the quantitatively determined amount of hydrogen peroxide. By using such an enzymatic reaction, it becomes possible to monitor a blood glucose level continuously with the sensor embedded in a living body.

As a method for embedding such a blood glucose level sensor in the subcutaneous tissue, for example, a method in which a sensor substrate to be used in the detection of a glucose level in the interstitial fluid is introduced into the subcutaneous tissue using an insertion needle for embedding this sensor substrate in the subcutaneous tissue, and thereafter the insertion needle is removed alone has been proposed (see, for example, JP-T-2002-503988 (Patent Document 1), JP-A-7-275227 (Patent Document 2), and JP-A-2005-279285 (Patent Document 3)).

However, in the method disclosed in Patent Document 1, the sensor substrate is only fitted in the insertion needle and engaged therewith, and therefore, the method has a problem that a trouble in which the sensor substrate easily comes off from the insertion needle occurs.

Further, in the method disclosed in Patent Document 2, a structure in which only a bent portion of the sensor substrate passes through a slit of the insertion needle, and the insertion needle can move (can slide) with respect to the sensor substrate only in the long axis direction (long side direction) is adopted, and further, in the method disclosed in Patent Document 3, a structure in which only a notch portion formed in the sensor substrate passes through a slit of the insertion needle, and the insertion needle can move (can slide) with respect to the sensor substrate only in the long axis direction (long side direction) is adopted. However, the bent portion and the notch portion each have a projection shape in the long axis direction, and therefore, when the insertion needle is pulled out, a trouble in which also the sensor substrate moves in the same direction as the pulling out direction due to a friction force between the projection and the slit may occur.

Due to the troubles as described above, when the insertion needle is removed, a resistance to the subcutaneous tissue is generated, and therefore, a problem that pain is given to a living body occurs.

### SUMMARY

An advantage of some aspects of the invention is to provide a sensor substrate capable of being placed in a living body without giving pain to the living body, and an analysis element, a glucose measurement device, and an insulin supply device, each of which includes this sensor substrate and has excellent reliability.

The advantage can be achieved by the configurations described below.

A sensor substrate according to an aspect of the invention is a sensor substrate, which is inserted into a living body by being guided by an insertion needle to be stuck and inserted into the living body, and includes a first region which is provided in a tip end portion of the sensor substrate and includes a detection electrode, a third region which includes a wiring section and has a smaller width than the width of a slit of the insertion needle, and a second region which is provided between the first region and the third region and has the same width as the width of the third region by gradually decreasing from the width of the first region.

According to this configuration, the slit of the insertion needle smoothly passes through the sensor substrate, so that the insertion needle can be detached from the sensor substrate, and the sensor substrate can be stably placed in the living body. In particular, the second region has a structure in which the width thereof gradually decreases, and therefore, the sensor substrate can be slid with respect to the insertion needle.

In the sensor substrate according to the aspect of the invention, it is preferred that the sensor substrate is formed with the same width in the first region and the third region.

According to this configuration, the slit of the insertion needle can be made to smoothly pass through the third region of the sensor substrate.

In the sensor substrate according to the aspect of the invention, it is preferred that the ratio of the width of the third region to the width of the first region is larger than 1/2 and smaller than 4/5.

According to this configuration, the slit of the insertion needle can be made to smoothly pass through the third region of the sensor substrate, and also the insertion needle can reliably guard the first region in the slit.

In the sensor substrate according to the aspect of the invention, it is preferred that the angle of the outer surfaces of the first region and the second region is 120° or more.

According to this configuration, the slit of the insertion needle can be made to smoothly pass through the third region of the sensor substrate.

In the sensor substrate according to the aspect of the invention, it is preferred that the sensor substrate includes two electrodes disposed in a parallel direction intersecting the long axis direction, and an electrode disposed in a series direction along the long axis direction of at least one of the electrodes in the first region.

According to this configuration, when the sensor substrate is inserted into a living body, pain given to the living body can be reduced, and also a glucose level can be detected with high accuracy.

An analysis element according to another aspect of the invention includes the sensor substrate according to the aspect of the invention and the insertion needle having a hollow section in which the sensor substrate is disposed.

According to this configuration, the slit of the insertion needle smoothly passes through the sensor substrate, so that the insertion needle can be detached from the sensor substrate, and the sensor substrate can be stably placed in the living body. In particular, the second region has a structure in which the width thereof gradually decreases, and therefore, the sensor substrate can be smoothly slid with respect to the insertion needle.

A glucose measurement device according to still another aspect of the invention includes the analysis element according to the aspect of the invention.

Such a glucose measurement device has excellent reliability.

An insulin supply device according to yet another aspect of the invention includes the glucose measurement device according to the aspect of the invention.

Such an insulin supply device has excellent reliability.

### BRIEF DESCRIPTION OF THE DRAWINGS

The invention will be described with reference to the accompanying drawings, wherein like numbers reference like elements.
FIG. 1 is a perspective view schematically showing an embodiment of a glucose measurement device according to the invention and shows a state where a detachable section included in an analysis element of the glucose measurement device is attached to a main body section.
FIG. 2 is a perspective view schematically showing the embodiment of the glucose measurement device according to the invention and shows a state where the detachable section included in the analysis element of the glucose measurement device is detached from the main body section.
FIG. 3 is a side view showing a state where the analysis element in the embodiment of the glucose measurement device according to the invention is attached to the skin.
FIG. 4 is a side view showing a state where a detection section included in the analysis element shown in FIG. 3 is inserted into a hollow section of a needle section included in the detachable section.
FIG. 5 is a plan view showing a state where a detection section included in the analysis element shown in FIG. 3 is inserted into the hollow section of the needle section included in the detachable section.
FIG. 6 is a vertical cross-sectional view taken along the line A-A shown in FIG. 5.
FIG. 7 is a plan view showing a first configuration of a positional relationship of electrodes in an electrode layer of the detection section included in the analysis element shown in FIG. 3.
FIG. 8 is a plan view for illustrating a method for detaching the detection section included in the analysis element shown in FIG. 3 from the hollow section of the needle section included in the detachable section.
FIG. 9 is a plan view for illustrating the size of the detection section included in the analysis element shown in FIG. 3.
FIG. 10 is a plan view for illustrating a positional relationship of the detection section included in the analysis element shown in FIG. 3 and the needle section included in the detachable section.
FIG. 11 is a plan view showing a second configuration of a positional relationship of electrodes in the electrode layer of the detection section included in the analysis element shown in FIG. 3.
FIG. 12 is a plan view showing a third configuration of a positional relationship of electrodes in the electrode layer of the detection section included in the analysis element shown in FIG. 3.
FIG. 13 is a plan view showing a fourth configuration of a positional relationship of electrodes in the electrode layer of the detection section included in the analysis element shown in FIG. 3.
FIG. 14 is a vertical cross-sectional view in a first region of the detection section included in the analysis element shown in FIG. 3.
FIG. 15 is a flowchart showing a method for measuring a glucose level using the glucose measurement device according to the invention.
FIG. 16 is a perspective view schematically showing a state where the analysis element according to the invention is attached to an insulin supply device.

### DESCRIPTION OF EXEMPLARY EMBODIMENTS

Hereinafter, a sensor substrate, an analysis element, a glucose measurement device, and an insulin supply device according to the invention will be described in detail based on embodiments shown in the accompanying drawings.

In this embodiment, a case where the glucose measurement device according to the invention is applied to a continuous glucose monitoring (CGM) device which continuously monitors a glucose level in the interstitial fluid over a long period of time will be described as an example.

### Glucose Measurement Device

First, a glucose measurement device (the glucose measurement device according to the invention) to which the analysis element according to the invention is attached will be described.

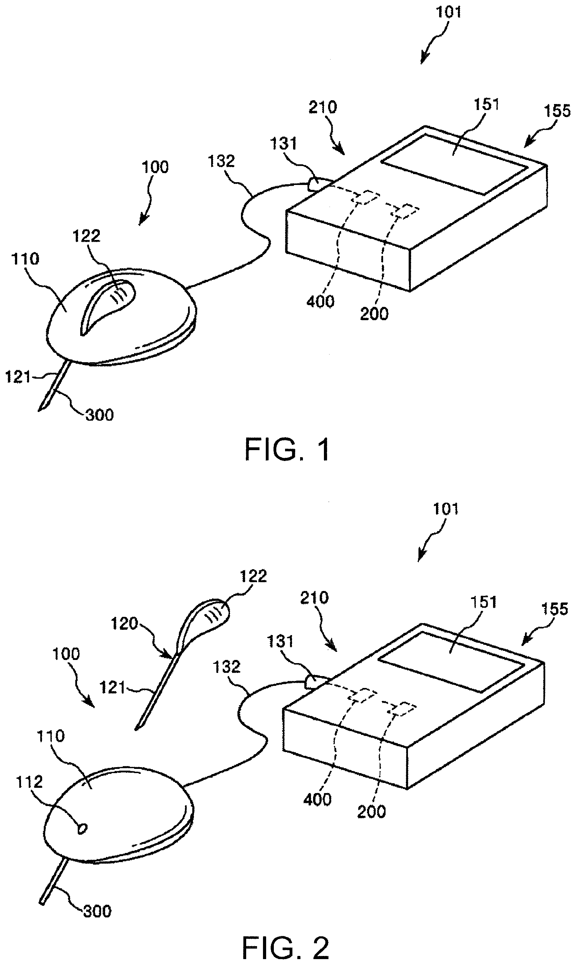

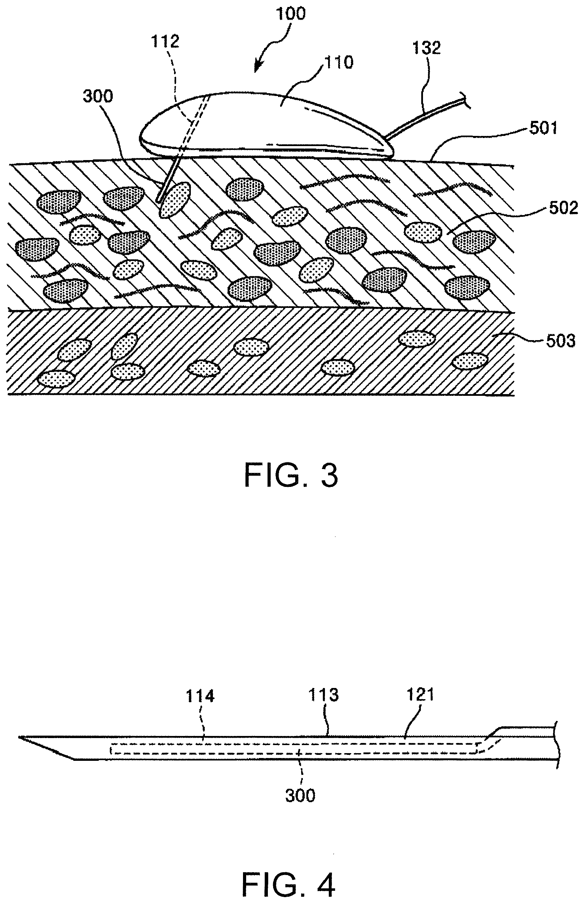

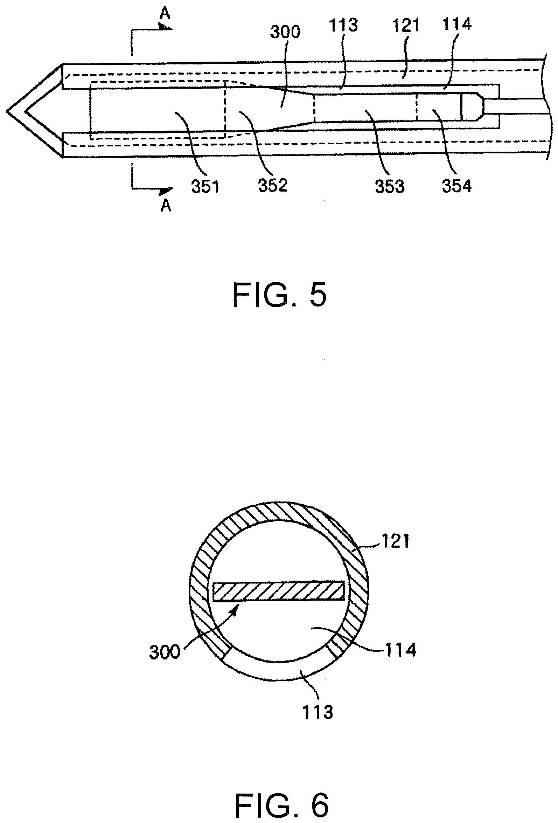

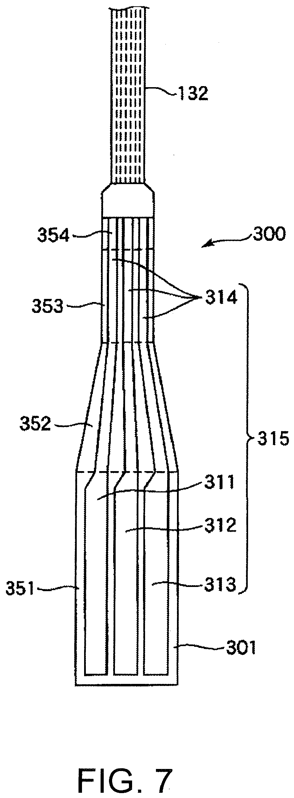

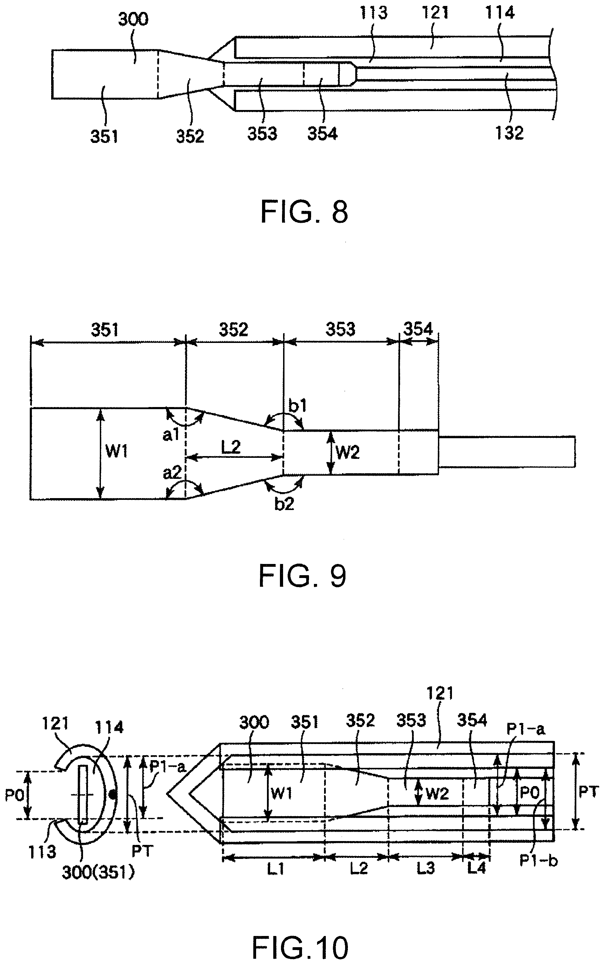

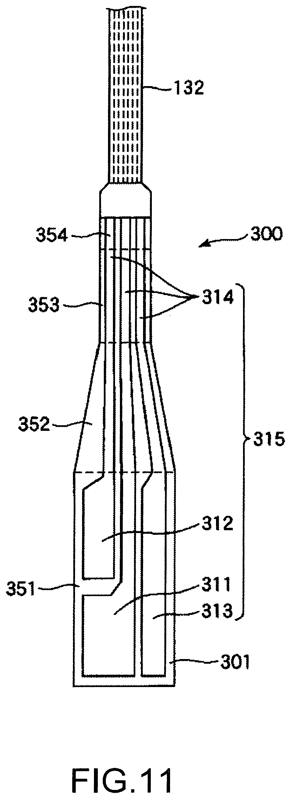

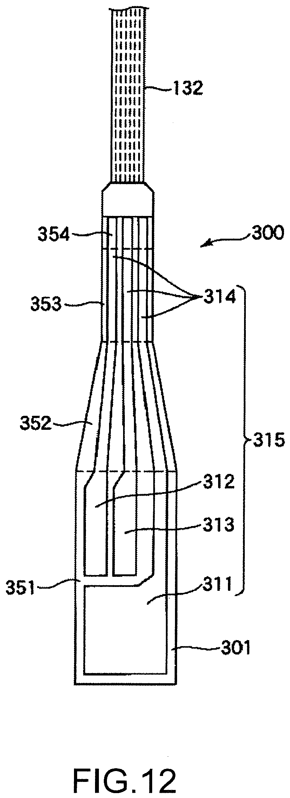

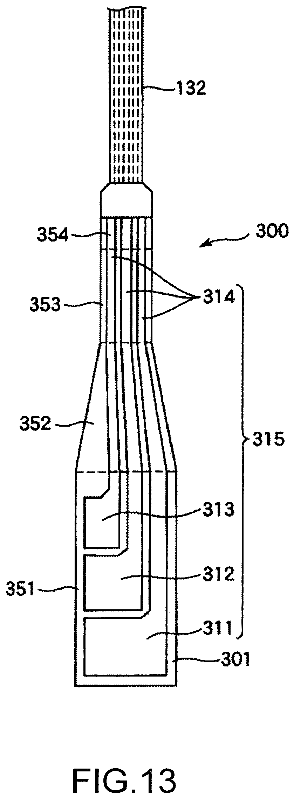

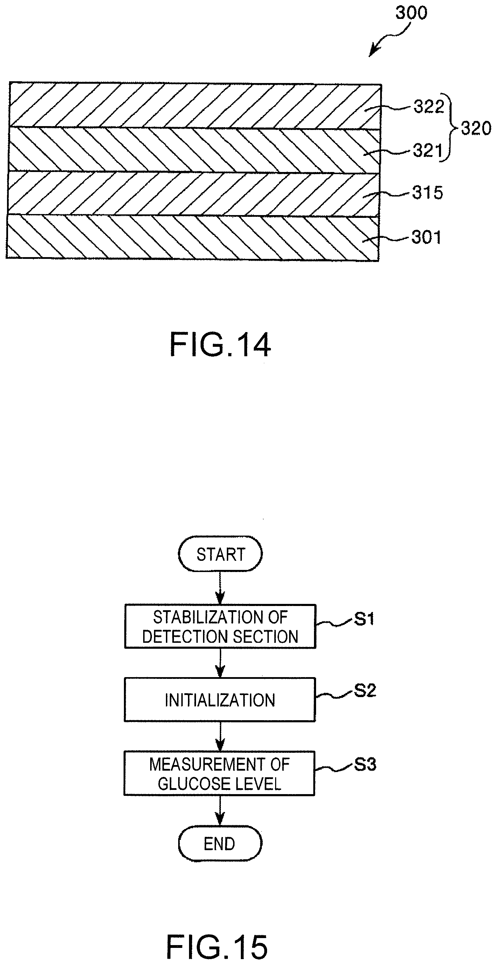

FIG. 1 is a perspective view schematically showing an embodiment of the glucose measurement device according to the invention and shows a state where a detachable section included in an analysis element of the glucose measurement device is attached to a main body section. FIG. 2 is a perspective view schematically showing the embodiment of the glucose measurement device according to the invention and shows a state where the detachable section included in the analysis element of the glucose measurement device is detached from the main body section. FIG. 3 is a side view showing a state where the analysis element in the embodiment of the glucose measurement device according to the invention is attached to the skin. FIG. 4 is a side view showing a state where a detection section included in the analysis element shown in FIG. 3 is inserted into a hollow section of a needle section included in the detachable section. FIG. 5 is a plan view showing a state where a detection section included in the analysis element shown in FIG. 3 is inserted into the hollow section of the needle section included in the detachable section. FIG. 6 is a vertical cross-sectional view taken along the line A-A shown in FIG. 5. FIG. 7 is a plan view showing a first configuration of a positional relationship of electrodes in an electrode layer of the detection section included in the analysis element shown in FIG. 3. FIG. 8 is a plan view for illustrating a method for detaching the detection section included in the analysis element shown in FIG. 3 from the hollow section of the needle section included in the detachable section. FIG. 9 is a plan view for illustrating the size of the detection section included in the analysis element shown in FIG. 3. FIG. 10 is a plan view for illustrating a positional relationship of the detection section included in the analysis element shown in FIG. 3 and the needle section included in the detachable section. FIG. 11 is a plan view showing a second configuration of a positional relationship of electrodes in the electrode layer of the detection section included in the analysis element shown in FIG. 3. FIG. 12 is a plan view showing a third configuration of a positional relationship of electrodes in the electrode layer of the detection section included in the analysis element shown in FIG. 3. FIG. 13 is a plan view showing a fourth configuration of a positional relationship of electrodes in the electrode layer of the detection section included in the analysis element shown in FIG. 3. FIG. 14 is a vertical cross-sectional view in a first region of the detection section included in the analysis element shown in FIG. 3. In the following description, in FIGS. 3, 4, 6, and 14, the upper side is referred to as "upper", and the lower side is referred to as "lower". Further, in FIGS. 5, and 7 to 13, the front side of the paper sheet is referred to as "upper", and the rear side of the paper sheet is referred to as "lower".

A glucose measurement device 101 shown in FIGS. 1 and 2 is used by connecting an analysis element 100 thereto, and includes the analysis element 100, a circuit 400 for obtaining an electric current value based on a glucose level in the analysis element 100, a calculation device 210 including a processing circuit 200 which analyzes the obtained electric current value, a display section 155 including a monitor 151 which displays a measurement value obtained by calculation by the calculation device 210, a connector 131 which attaches (connects) the analysis element 100 to the display section 155, and a wiring 132 which connects the analysis element 100 to the connector 131.

As shown in FIGS. 1 to 4, the analysis element (detection element) 100 includes a main body section 110 which includes a detection section (sensor substrate) 300 to be inserted into a subcutaneous tissue 502 and a detachable section 120 which can be attached to and detached from the main body section 110 and includes a needle section (insertion needle) 121 on a tip end side.

The detachable section 120 includes a holding section 122 located on a base end side and the needle section 121 located on a tip end side, and is attached to the main body section 110 by inserting the needle section 121 into a through-hole 112 provided in the main body section 110 (see FIG. 1), and further can be detached by pulling out the needle section 121 in a state where the holding section 122 is held (see FIG. 2).

The needle section (insertion needle) 121 is sharp at the tip end, and includes a hollow section 114 which opens on the tip end side, and has a cylindrical shape as a whole. Then, the needle section 121 includes a slit 113 which opens the hollow section 114 from the middle part to the tip end on the side surface thereof.

When the detachable section 120 including such a needle section 121 is attached to the main body section 110, and made to pass through the through-hole 112 and protrude from the lower surface of the main body section 110, as shown in FIGS. 4 to 6, the detection section 300 is configured to be inserted (disposed) inside the hollow section 114. Therefore, when the main body section 110 is attached to the epidermis 501, the needle section 121 is stuck into the epidermis (living body) 501. However, at this time, the detection section 300 inserted into the hollow section 114 of the needle section 121 is also stuck into the epidermis 501 along with the needle section 121 and is inserted into the subcutaneous tissue (living body) 502. At the time of this insertion, the detection section 300 is disposed in the hollow section 114, and is in a state of being surrounded by the needle section 121, and therefore, even if an external force to bend the needle section 121 is applied, the needle section 121 exhibits a function as a buffer material, and thus, it is possible to properly suppress or prevent the detection section 300 from being bent.

Then, after the needle section 121 is inserted into the subcutaneous tissue 502 in a state where the detection section 300 is disposed in the hollow section 114, the detachable section 120 is detached from the main body section 110 by holding the holding section 122, whereby the needle section 121 is removed (pulled out) alone from the subcutaneous tissue 502 in a state where the detection section 300 is inserted (left) in the subcutaneous tissue 502. In this manner, when the main body section 110 is attached to the epidermis 501, the detachable section 120 (needle section 121) is used as a guiding member for percutaneously disposing the detection section 300 protruding from the main body section 110 in the subcutaneous tissue 502.

In this embodiment, the shape of the cross section of the needle section 121 is a true circle as shown in FIG. 6, however, the shape of the cross section thereof is not particularly limited as long as the needle section 121 includes the hollow section 114 capable of disposing the detection section 300 therein, and may be, for example, a polygon such as a triangle or a tetragon other than an ellipse. However, by forming the needle section 121 to have a cross-sectional shape of a circle such as a true circle or an ellipse, even if an external force is applied to the detection section 300, the detection section 300 can be uniformly held by the wall surface of the needle section 121, and thus, it is possible to properly suppress or prevent the detection section 300 from being locally bent.

Further, the detachment of the detachable section 120 from the main body section 110, that is, the detachment of the detection section 300 from the hollow section 114 is performed without giving pain to a measurer (living body) depending on a relationship between the shapes of the needle section 121 and the detection section 300, however, a detailed description thereof will be made later.

The main body section 110 has a dome shape as a whole and includes the detection section 300 protruding from the lower surface thereof. This main body section 110 includes an adhesive layer on the lower surface and is attached (fixed) to the epidermis 501 by bringing this lower surface into contact with the epidermis 501. At this time, the detection section 300 is placed in the subcutaneous tissue 502 by being guided by the needle section 121 described above. That is, the detection section 300 is guided by the needle section (insertion needle) 121 and is inserted into the subcutaneous tissue (into the living body) 502.

The detection section 300 (the sensor substrate according to the invention) has a strip shape as a whole and includes a first region 351 provided in a tip end portion, a third region 353 provided in a base end portion (on the wiring 132 side), and a second region provided between the first region 351 and the third region 353 in a plan view. These first region 351 to the third region 353 constitute a placement region to be placed (inserted) in the subcutaneous tissue 502, and further, the detection section 300 has a fourth region 354 which is provided nearer to the base end side than the third region 353 and is not placed in the subcutaneous tissue 502. A wiring 314 is electrically connected to the wiring 132 through this fourth region 354.

As shown in FIGS. 5 and 10, the first region 351 has a width W1 which is thinner (narrower) than the width PT of the hollow section 114 of the needle section 121 and also broader (wider) than the width P0 of the slit 113 of the needle section 121. According to this, when the detection section 300 is disposed in the hollow section 114, particularly, when the needle section 121 is inserted into the subcutaneous tissue 502, the detection section 300 is reliably prevented from being detached from the needle section 121 through the slit 113. Therefore, it is possible to insert the needle section 121 into the subcutaneous tissue 502 without giving pain caused by the detachment of the detection section 300 to a measurer.

Further, when a distance from one end portion of the slit 113 to the inner surface of the hollow section 114 on the other facing side in the short side direction is denoted by P1-a, and a distance from the other end portion of the slit 113 to the inner surface of the hollow section 114 on one facing side in the short side direction is denoted by P1-b, in this embodiment, as shown in FIG. 10, the following relationship: P1-a = P1-b (= P1) in which these distances are the same is satisfied, however, at this time, it is preferred that the following relationship: W1 > P1 is satisfied. The detection section 300 can be more reliably prevented from being detached from the needle section 121 through the slit 131.

Further, as shown in FIGS. 5, 8, and 10, the third region 353 has a width W2 which is thinner (smaller) than the width P0 of the slit 113 of the needle section 121. According to this, when the needle section 121 is removed (pulled out) from the subcutaneous tissue 502, as shown in FIG. 8, by pulling out the needle section 121 to a position corresponding to the third region 353, the third region 353 (detection section 300) located in the hollow section 114 can be detached from the needle section 121 through the slit 113. That is, the needle section 121 is shifted until the first region 351 and the second region 352 are located outside the hollow section 114, and thereafter, the third region 353 is moved outside the hollow section 114 through the slit 113 , whereby the detection section 300 can be detached from the needle section 121.

The width W2 of the third region 353 may be any width as long as it is thinner than the width P0 of the slit 113, however, it is preferred that the following relationship: 0.2 ≤ W2/P0 ≤ 0.9 is satisfied, and it is more preferred that the following relationship: 0.4 ≤ W2/P0 ≤ 0.8 is satisfied. According to this, the detachment of the detection section 300 from the hollow section 114 through the slit 113 in the third region 353 can be more smoothly performed, and also the occurrence of a break in the third region 353 can be reliably prevented.

Further, the second region 352 has a width which is the same as the width of the first region 351 on the tip end side and gradually decreases from the tip end side to the base end side from the width of the first region 351 and is the same as the width of the third region 353 on the base end side. Since the second region 352 having a width which gradually decreases as described above is included, it is possible to properly suppress or prevent the detection section 300 from being locked by the slit 113 when the detection section 300 is pulled out from the hollow section 114 until the detection section 300 is located at a position corresponding to the third region 353 as shown in FIG. 8 from a state where the entire detection section 300 including the first region 351 is disposed in the hollow section 114 as shown in FIG. 5. Due to this, as shown in FIG. 8, the detection section 300 can be smoothly slid (moved) to a state where the first region 351 and the second region 352 are pulled out from the hollow section 114, and the third region 353 is disposed alone in the hollow section 114. In this manner, the detection section 300 can be smoothly moved with respect to the needle section 121. As a result, it is possible to properly suppress or prevent pain from being given to a measurer accompanying this movement.

Further, in this embodiment, the first region 351 and the third region 353 both have a rectangular shape in a plan view and are formed with the same width in each region, and the width of the first region 351 is larger than the width of the third region 353. According to this, as shown in FIG. 8, the detachment of the third region 353 located in the hollow section 114 from the needle section 121 through the slit 113 can be more smoothly performed. That is, the third region 353 of the detection section 300 can be made to smoothly pass through the slit 113.

Further, in this case, it is preferred that the ratio of the width W2 of the third region 353 to the width W1 of the first region 351 (W2/W1) is larger than 1/2 and smaller than 4/5. That is, it is preferred that the following relationship: 1/2 < W2/W1 < 4/5 is satisfied, it is more preferred that the following relationship: 1/3 < W2/W1 < 4/5 is satisfied, and it is further more preferred that the following relationship: 1/2 < W2/W1 < 4/5 is satisfied. According to this, as shown in FIG. 5, when the detection section 300 is disposed in the hollow section 114, the first region 351 is reliably guarded by the slit 113, and the detection section 300 can be more reliably prevented from being detached from the needle section 121 through the slit 113. Further, as shown in FIG. 8, after the needle section 121 is pulled out to a position corresponding to the third region 353, the third region 353 located in the hollow section 114 can be slid in the slit 113, and therefore, the detection section 300 can be more reliably detached from the needle section 121. Further, in the third region 353 whose strength decreases when the detection section 300 is detached from the needle section 121, the detection section 300 can be properly suppressed or prevented from breaking.

The width of the fourth region 354 is not particularly limited, and is set to, for example, preferably 10 mm or less, more preferably 3 mm or more and 5 mm or less. According to this, in the third region 353, when the detection section 300 is detached from the needle section 121 through the slit 113, also this fourth region 354 can be made to exhibit the same function as that of the third region 353.

Further, angles a1 and a2 formed by the side surface facing the inner surface of the needle section 121 of the first region 351 and the side surface facing the inner surface of the needle section 121 of the second region 352 on the central axis side, that is, the angles a1 and a2 of the outer surfaces of the first region 351 and the second region 352 are each preferably 120° or more and less than 180°, more preferably 135° or more and less than 175°, further more preferably 150° or more and less than 170°. In addition, angles b1 and b2 formed by the side surface facing the inner surface of the needle section 121 of the second region 352 and the side surface facing the inner surface of the needle section 121 of the third region 353 on the opposite side to the central axis, that is, the angles b1 and b2 of the outer surfaces of the second region 352 and the third region 353 are each preferably 120° or more and less than 180°, more preferably 135° or more and less than 175°, further more preferably 150° or more and less than 170°. When the angles a1 and a2 and the angles b1 and b2 are within the above ranges, the detachment of the first region 351 and the second region 352 of the detection section 300 from the hollow section 114 can be smoothly performed. As a result, the third region 353 of the detection section 300 can be made to smoothly pass through the slit 113. Further, it is preferred that the angles a1 and a2 and the angles b1 and b2 are the same angle. According to this, the symmetry of the detection section 300 is ensured, and the production of the detection section 300 can be easily performed.

The width W2 of the third region 353 may be any width as long as it is thinner than the width P0 of the slit 113 of the needle section 121, and it is not necessary to have a rectangular shape in a plan view or to have a constant width W2 in the third region 353 as shown in FIGS. 5, and 7 to 10, and the width W2 may gradually decreases from the tip end side to the base end side in the same manner as the second region 352. That is, the second region 352 and the third region 353 may be integrally formed such that the width of these regions gradually and continuously decreases. It can also be said that in the detection section 300 having such a shape, the formation of the third region 353 is omitted, and the second region 352 have both functions of the second region and the third region.

The interstitial fluid contained in the subcutaneous tissue 502 by transferring from the blood 503 to the subcutaneous tissue 502 comes into contact with the detection section 300 having such a shape. Due to this, by the detection section 300, glucose in the interstitial fluid is detected. Then, by attaching the main body section 110 to the epidermis 501, the detection section 300 can be placed in the subcutaneous tissue 502 over a long period of time. Therefore, the detection of glucose in the interstitial fluid can be continuously performed. That is, the main body section 110 (analysis element 100) is used in a CGMS (continuous glucose monitoring system) which continuously observes a glucose level in the interstitial fluid.

Here, the detection of glucose by the detection section 300 is performed using a principle as described below.

That is, by using an enzyme, glucose can be quantitatively determined. As the enzyme, glucose oxidase, glucose dehydrogenase, or the like can be used. For example, in the presence of glucose oxidase, when glucose and oxygen are present in the vicinity of the enzyme, gluconic acid and hydrogen peroxide are produced by an enzymatic reaction as shown in the following formula (1). Then, by measuring the amount of an electric current generated by electrical decomposition by applying a voltage (for example, 600 mV) to the produced hydrogen peroxide between a working electrode and a counter electrode, the amount of hydrogen peroxide can be quantitatively determined. Therefore, based on this quantitatively determined amount of hydrogen peroxide, a glucose level can be calculated.

When hydrogen peroxide is electrically decomposed, on a working electrode (anode) side, protons, oxygen, and electrons are generated by the electrical decomposition of hydrogen peroxide as shown in the following formula (2), and on a counter electrode (cathode) side, hydroxide ions are generated by reacting electrons supplied from the working electrode, and oxygen and water present in the vicinity of the electrode with one another as shown in the following formula (3).
Enzymatic reaction:

   glucose + O₂ + H₂O → gluconic acid + H₂O₂ (1)
Working electrode:

   H₂O₂ → O₂ + 2H⁺ + 2e⁻ (2)
Counter electrode:

   O₂ + 2H₂O + 4e⁻ → 4OH⁻ (3)

Hereinafter, the respective sections of the detection section 300 which detects glucose using such a principle will be described in detail.

As shown in FIG. 14, the detection section 300 includes a substrate 301, an electrically conductive layer (electrode) 315, and a sensing layer (enzyme layer) 320.

The substrate (base substrate) 301 supports the respective components (in this embodiment, the electrically conductive layer 315 and the sensing layer 320) constituting the detection section 300.

As the constituent material of the substrate 301, a variety of materials can be used without being particularly limited as long as the material does not chemically react with air, water, and body fluids such as blood and interstitial fluid, and is stable. Specific examples thereof include inorganic materials such as glass and SUS, and resin materials such as amorphous polyarylate (PAR), polysulfone (PSF), polyethersulfone (PES), polyphenylene sulfide (PPS), polyether ether ketone (PEEK, another name: aromatic polyether ketone), polyimide (PI), polyetherimide (PEI), fluororesins (fluorocarbon polymers), nylon, polyamides (PA) including amides, and polyesters such as polyethylene terephthalate (PET), and among these, it is possible to use one type or two or more types in combination.

The electrically conductive layer (electrode layer) 315 detects electrons generated by electrically decomposing hydrogen peroxide produced in the below-mentioned sensing layer 320, and measures the detected electrons as an electric current amount.

This electrically conductive layer 315 is formed on the substrate 301 and includes a working electrode 311, a counter electrode 312, a reference electrode 313, and a wiring 314.

The respective electrodes 311, 312, and 313 are independently electrically connected to the circuit 400 and the processing circuit 200 through the wiring 314, the wiring 132, and the connector 131. According to this, an electric current value measured by the respective electrodes 311 and 312 (electrically conductive layer 315) is transmitted to the circuit 400 and the processing circuit 200 included in the display section 155 through the wirings 314 and 132, and a glucose level in the interstitial fluid is calculated as a measurement value by an analysis by the calculation device 210 including the processing circuit 200. Then, this measurement value (glucose level) is displayed on the monitor 151, and thus, a wearer is continuously informed of the glucose level.

As shown in FIG. 7, the respective electrodes (detection electrodes) 311, 312, and 313 are formed in the first region 351, and are each electrically connected to the wiring (wiring section) 132 through the wiring 314 formed in the second region 352 and the third region 353.

Further, the respective three electrodes 311, 312, and 313 formed in the first region 351 are disposed side by side in this order along the short side direction (series direction) orthogonal to the long side direction (long axis direction). According to this, it shows a tendency that the width of the detection section 300 slightly increases, however, the length of the detection section can be decreased, and therefore, an advantage that it is not necessary to insert the detection section 300 into a deep portion of the subcutaneous tissue 502 can be obtained. Further, a glucose level in the interstitial fluid in a deep portion, which is uniform, can be measured, and therefore, the glucose level is detected with higher accuracy.

Incidentally, as the configuration of the respective electrodes 311, 312, and 313 formed in the first region 351, other than the first configuration in which the three electrodes are disposed side by side in the short side direction, any of the second to fourth configurations shown in FIGS. 11 to 13 may be adopted.

That is, in the second configuration shown in FIG. 11, on the tip end side of the first region 351, the working electrode 311 and the reference electrode 313 are disposed side by side in the short side direction, and on the base end side of the first region 351, the counter electrode 312 is disposed. In other words, in the first region 351, the two electrodes 311 and 313 disposed in a parallel direction intersecting the long axis direction, and the electrode 312 disposed in a series direction along the long axis direction with respect to the working electrode 311 are provided. According to this, as compared with the first configuration and the fourth configuration, the detection section 300 can be made to have an average width and an average length.

In the third configuration shown in FIG. 12, on the tip end side of the first region 351, the working electrode 311 is disposed, and on the base end side of the first region 351, the counter electrode 312 and the reference electrode 313 are disposed side by side in the short side direction. According to this, as compared with the first configuration and the fourth configuration, the detection section 300 can be made to have an average width and an average length.

In the fourth configuration shown in FIG. 13, the respective three electrodes 311, 312, and 313 are disposed side by side in this order from the tip end side along the long side direction. According to this, it shows a tendency that the length of the detection section 300 slightly increases, however, the width of the detection section can be decreased, and therefore, an advantage that pain given to a living body when the detection section 300 is inserted into the subcutaneous tissue 502 can be further reduced can be obtained.

In light of the above, by using the detection section 300 having any of the second to fourth configurations, pain given to a living body when the detection section 300 is inserted into the subcutaneous tissue 502 can be reduced. Further, by using the detection section 300 having any of the first to third configurations, a glucose level can be detected with high accuracy.

Incidentally, the first to fourth configurations, and also the areas, lengths, widths, and the like of the respective electrodes 311, 312, and 313 included therein can be appropriately selected according to the measurement accuracy or the like required for the glucose measurement device.

Further, the positions of the respective electrodes 311, 312, and 313 in the first to fourth configurations are not limited to those shown in the drawings, and the respective electrodes 311, 312, and 313 can be disposed at arbitrary positions.

Further, the sizes of the respective electrodes 311, 312, and 313 are not particularly limited, however, for example, the width (short side) of each of the working electrode 311 and the counter electrode 312 is preferably about 0.1 mm or more and 0.3 mm or less, more preferably about 0.2 mm. The length (long side) thereof is preferably about 0.1 mm or more and 3.0 mm or less, more preferably about 2.0 mm.

The working electrode 311 and the counter electrode 312 having such a size may have the same area or different areas, however, it is preferred that the following relationship: the area of the counter electrode 312 > the area of the working electrode 311 is satisfied. This is because it is necessary to use up the amount of electrochemically transferred electrons in the working electrode 311 by a reverse reaction in the counter electrode 312, and therefore, by satisfying the above relationship, the stabilization of the reverse reaction is achieved.

The width (short side) of the reference electrode 313 is preferably about 0.01 mm or more and 0.2 mm or less. Further, the length (long side) thereof is preferably about 0.1 mm or more and 3.0 mm or less.

The reference electrode 313 having such a size preferably has an area which is 20% or more and 100% or less with respect to the working electrode 311 and the counter electrode 312. The reference electrode 313 is not used for detecting an electric current value to be measured for calculating a glucose level, and therefore, the area thereof may be smaller than those of the working electrode 311 and the counter electrode 312. However, in order to prevent the decrease in the characteristics due to adhesion of protein or the like contained in the interstitial fluid when it is placed in the subcutaneous tissue 502, it is preferred that the reference electrode 313 has an area in the above range.

The constituent materials of the respective electrodes 311, 312, and 313 are not particularly limited as long as they can be used as an enzyme electrode, and examples thereof include metal materials such as gold, silver, platinum, and alloys containing these metals, metal oxide-based materials such as ITO, and carbon-based materials such as carbon (graphite).

The deposition of the respective electrodes 311, 312, and 313 can be performed by a sputtering method, a plating method, or a vacuum heating vapor deposition method in the case where the respective electrodes 311, 312, and 313 are constituted by platinum, gold, or an alloy thereof. Further, in the case where the respective electrodes 311, 312, and 313 are constituted by carbon graphite, the deposition can be achieved by mixing carbon graphite in a binder dissolved in a suitable solvent and applying the resulting mixture.

The sensing layer 320 is formed by being stacked on the electrically conductive layer 315, and hydrogen peroxide is produced by an enzymatic reaction from glucose permeating from the interstitial fluid coming into contact with the upper surface of the sensing layer 320, and then, the produced hydrogen peroxide is supplied to the above-mentioned electrically conductive layer 315. The sensing layer 320 senses glucose by the enzymatic reaction in which hydrogen peroxide is produced from glucose.

In this embodiment, this sensing layer 320 includes an analyte sensing layer 321 and an analyte control layer 322 as shown in FIG. 14, and is constituted by a stacked body in which these layers are stacked in this order from the electrically conductive layer 315 side. Hereinafter, the respective layers will be described.

The analyte sensing layer (enzyme layer) 321 is formed so as to cover the working electrode 311, the counter electrode 312, and the reference electrode 313, and produces hydrogen peroxide by an enzymatic reaction from glucose permeating through the analyte control layer 322. Then, the analyte sensing layer 321 supplies the produced hydrogen peroxide to the electrically conductive layer 315.

This analyte sensing layer 321 is a layer configured to contain an enzyme, and as described above, the analyte element 100 detects (senses) glucose in the interstitial fluid, and therefore, as the enzyme, glucose oxidase (GOD) is preferably used. By using glucose oxidase, the enzymatic reaction represented by the above formula (1) can be made to proceed with high activity, and therefore, hydrogen peroxide can be reliably produced from glucose in the presence of O₂ and H₂O.

Further, in the analyte sensing layer 321, other than the enzyme, a resin material is contained for the purpose of retaining the enzyme in the analyte sensing layer 321.

The resin material is not particularly limited, but, for example, methyl cellulose (MC), acetyl cellulose (cellulose acetate), polyvinylpyrrolidone (PVP), polyvinyl alcohol (PVA), a polyvinyl alcohol-polyvinyl acetate copolymer (PVA-PVAc), or the like is preferably used, and among these, it is possible to use one type or two or more types in combination. By using these resin materials, the enzyme is retained in the analyte sensing layer 321, and the transfer of the enzyme to the outside of the analyte sensing layer 321 can be suppressed.

Further, in the analyte sensing layer 321, a binder or a curing agent, and other than this, albumin, a phosphate buffer, or the like may be contained.

Examples of the binder or the curing agent include a material having two or more functional groups such as aldehyde and isocyanate in the molecule. By including such a binder or a curing agent in the analyte sensing layer 321, the analyte sensing layer 321 can retain the enzyme in the analyte sensing layer 321 at a high retention ratio.

Specific examples of the binder or the curing agent include glutaraldehyde, toluene diisocyanate, and isophorone diisocyanate, and among these, it is possible to use one type or two or more types in combination. Further, examples of the binder or the curing agent utilizing UV curability include a poly(vinyl alcohol)-styrylpyridinium compound (PVA-SbQ).

The analyte sensing layer 321 containing such a binder or a curing agent can be obtained by curing a resin composition in which the binder or the curing agent, a resin material having a functional group capable of binding to a functional group contained in the binder or the curing agent, specifically, a hydroxy group, an amino group, an epoxy group, or the like at a terminal end, and the enzyme are mixed with one another.

In the case where a poly (vinyl alcohol)-styrylpyridinium compound (PVA-SbQ) is used as the binder or the curing agent utilizing UV curability, the addition of a resin material having a functional group at a terminal end is omitted, and the analyte sensing layer 321 can also be constituted by a cured material obtained by curing a resin composition containing PVA-SbQ and the enzyme. In this case, in the analyte sensing layer 321, the enzyme is retained in a porous body constituted by PVA-SbQ, and the interstitial fluid permeating from the analyte control layer 322 side can be smoothly incorporated in the analyte sensing layer 321, and the opportunity to contact glucose contained in the interstitial fluid with the enzyme can be increased, so that the enzymatic reaction represented by the above formula (1) can be made to smoothly proceed, and thus, hydrogen peroxide can be reliably produced from glucose in the presence of O₂ and H₂O. In addition, since the enzyme can be firmly retained in the porous body constituted by PVA-SbQ, the transfer of the enzyme to the analyte control layer 322 side can be properly suppressed or prevented.

Further, examples of the albumin include human albumin and bovine albumin, and by including albumin, the protection and stabilization of the enzyme can be achieved.

The average thickness of such an analyte sensing layer 321 is not particularly limited, but is preferably about 0.1 µm or more and 10 µm or less, more preferably about 0.5 µm or more and 5.0 µm or less. According to this, hydrogen peroxide can be smoothly produced by an enzymatic reaction between glucose permeating from the analyte control layer 322 side and the enzyme retained in the analyte sensing layer 321, and also the produced hydrogen peroxide can be smoothly supplied to the electrically conductive layer 315.

The analyte control layer 322 is stacked on the upper side of the analyte sensing layer 321, and by this analyte control layer 322, while suppressing or preventing the analyte sensing layer 321 from coming into contact with the measurement target (interstitial fluid, and moreover, blood), a function to allow glucose to penetrate and smoothly permeate into the analyte sensing layer 321 is exhibited. Further, also a function as a blocking layer which prevents the leakage of the enzyme retained in the analyte sensing layer 321 to the interstitial fluid side is exhibited.

Further, it is preferred that the analyte control layer 322 having such a function is capable of allowing oxygen to penetrate (permeate) more than glucose. According to this, in the detection of glucose using the reactions shown in the above formulae (1) to (3), an apparent decrease in the measurement value of glucose due to a lack of oxygen can be properly suppressed or prevented. That is, the improvement of the detection accuracy of the measurement value of glucose can be achieved.

Such an analyte control layer 322 is not particularly limited, however, for example, a layer in which a cross-linked structure is constructed by forming a urethane bond using a cross-linking agent such as an isocyanate compound and also using polyethylene glycol (PEG), polypropylene glycol (PPG), 4-hydroxybutyl acrylate, and the like, each of which is a polymer having a terminal hydroxy group, alone or in admixture is particularly preferably used. According to this, the above-described function as the analyte control layer 322 can be more remarkably exhibited.

Further, other than this, a layer constituted by aminopropyl polysiloxane or the like, in which a urea resin is formed using isocyanate and an amino group, can be used, and moreover, a layer constituted by a siloxane resin such as polydimethylsiloxane can also be used.

The average thickness of such an analyte control layer 322 is not particularly limited, but is preferably about 0.1 µm or more and 10 µm or less, more preferably about 0.5 µm or more and 5. 0 µm or less. According to this, glucose can be made to smoothly permeate into the analyte control layer 322, and also the leakage of the enzyme retained in the analyte sensing layer 321 to the interstitial fluid side can be property suppressed of prevented.

The sensing layer 320 may include another layer other than the above-mentioned analyte sensing layer 321 and analyte control layer 322, and examples of such a layer include a noise removal layer which is stacked on the lower side of the analyte sensing layer 321 and an intermediate layer or the like to be formed between the analyte sensing layer 321 and the analyte control layer 322.

The noise removal layer is provided for preventing the decrease in the detection sensitivity for the measurement value of glucose caused by penetration of a compound such as acetaminophen, ascorbic acid, or uric acid, which may be contained in the interstitial fluid, through the analyte sensing layer 321 to reach the electrically conductive layer 315.

Further, the intermediate layer is provided for exhibiting a function as a barrier layer for preventing the transfer of the enzyme to the analyte control layer 322 side.

Incidentally, in the detection section 300, all of the working electrode 311, the counter electrode 312, the reference electrode 313, and the wiring 314 included in the electrically conductive layer 315, that is, all of the layers from the first region 351 to the third region 353 may be covered by the analyte sensing layer 321, or all of the working electrode 311, the counter electrode 312, and the reference electrode 313, that is, all the first region 351 may be covered, or further, the working electrode 311 and the counter electrode 312 may be selectively covered, or the working electrode 311 may be selectively covered.

Further, as shown in FIGS. 1 and 2, the display section 155 includes the circuit 400 for driving the detection section 300, which is disposed therein and electrically connected thereto through the wirings 314 and 132.

To this circuit 400, the working electrode 311, the reference electrode 313, and the counter electrode 312 included in the detection section 300 are electrically connected.

By operating the circuit 400, a voltage difference of, for example, 600 mV is maintained between the working electrode 311 and the reference electrode 313, and this difference becomes an applied voltage to the detection section 300, and as a result, electrons are produced by electrically decomposing hydrogen peroxide contained in the sensing layer 320. Then, the electrons are measured by the circuit 400 as an electric current value based on a glucose level between the working electrode 311 and the counter electrode 312, and also this electric current value is analyzed by the calculation device 210 included in the processing circuit 200, whereby a glucose level is calculated as a measurement value, and further, this measurement value is displayed on the monitor 151.

### Measurement Method

The measurement of a glucose level in the interstitial fluid using the above-mentioned glucose measurement device 101 is specifically performed, for example, as follows.

FIG. 15 is a flowchart showing a method for measuring a glucose level using the glucose measurement device according to the invention.
[1] First, the detection section 300 is inserted into the subcutaneous tissue 502 and stabilized by bringing the detection section 300 (sensing layer 320) into contact with the interstitial fluid (S1).
[2] Subsequently, a fixed voltage is applied between the working electrode 311 and the reference electrode 313 for a predetermined period of time. By doing this, hydrogen peroxide generated in the vicinity of the working electrode 311 of the sensing layer 320 during stabilization is electrically decomposed as shown in the above formula (2), whereby, the vicinity of the working electrode 311 of the sensing layer 320 is initialized (S2).
[3] Subsequently, after an interval of a fixed period of time, a fixed voltage is applied between the working electrode 311 and the reference electrode 313 for a predetermined period of time. By doing this, hydrogen peroxide generated in the vicinity of the working electrode 311 of the sensing layer 320 is electrically decomposed as shown in the above formula (2), and electrons generated as a result of the electrical decomposition are measured as the value of an electric current flowing between the working electrode 311 and the counter electrode 312, whereby a glucose level in the interstitial fluid is obtained (S3).

This step [3] may be performed once, however, by performing the step [3] repeatedly, and obtaining an average of the glucose levels measured during this period, the glucose levels are averaged, and thus, the glucose level can be obtained with higher reliability.

By performing the step [1] to step [3] as described above repeatedly at intervals of a fixed time, a glucose level (glucose concentration) in the interstitial fluid can be continuously measured.

### Insulin Supply Device

The analysis element according to the invention is not only attached to a glucose measurement device as described above, but also attached to, for example, an insulin supply device (the insulin supply device according to the invention) .

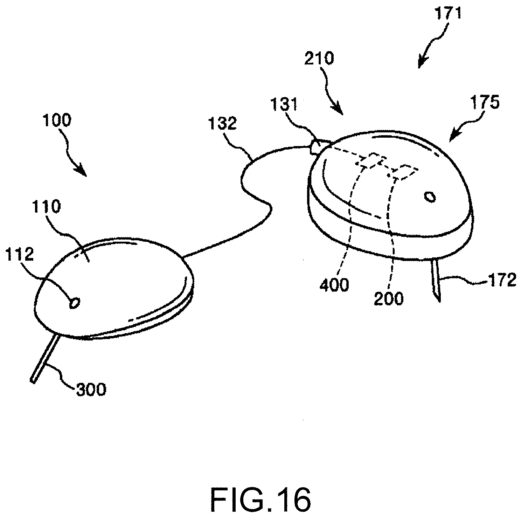

FIG. 16 is a perspective view schematically showing a state where the analysis element according to the invention is attached to an insulin supply device.

An insulin supply device (insulin pump) 171 shown in FIG. 16 is used by connecting an analysis element 100 thereto, and includes the analysis element 100 including a detection section 300, a calculation device 210 including a processing circuit 200 which analyzes an electric current value obtained by the analysis element 100, a supply section 175 which includes a needle section 172 and supplies (administers) insulin to a subcutaneous tissue 502 based on a measurement value obtained by calculation by the calculation device 210, a connector 131 which attaches (connects) the analysis element 100 to the supply section 175, and a wiring 132 which connects the processing circuit 200 to the connector 131. In such an insulin supply device 171, the value of an electric current flowing between a working electrode 311 and a counter electrode 312 is transmitted to the processing circuit 200 through a circuit 400 included in a main body section 110 and the wiring 132, and a glucose level (glucose concentration) in an interstitial fluid is calculated as a measurement value by an analysis by the calculation device 210 including the processing circuit 200. Then, based on this measurement value (glucose level), that is, in the case where the measurement value is higher than a preset concentration, the insulin supply device 171 is operated, and insulin is automatically administered to a wearer through the needle section 172.

Hereinabove, the sensor substrate, the analysis element, the glucose measurement device, and the insulin supply device according to the invention have been described with reference to the embodiments shown in the drawings, however, the invention is not limited thereto.

For example, the configurations of the respective components in the sensor substrate, the analysis element, the glucose measurement device, and the insulin supply device according to the invention can be replaced with an arbitrary configuration having a similar function. In addition, another arbitrary configuration may be added to the invention. Further, the invention may be configured such that arbitrary two or more configurations (features) of the above-mentioned glucose measurement device and insulin supply device are combined.

Further, the detection section 300 is not only inserted into the subcutaneous tissue, but also may be inserted into the skin.

Further, in the glucose measurement device and the insulin supply device, an electric current value measured by the analysis element may be transmitted to the processing circuit without passing through the wiring, and for example, an electric current value may be transmitted wirelessly to the processing circuit through a communication unit.

Further, the glucose measurement device is not limited to a glucose measurement device in which an analysis element and a display section are connected to each other through a wiring, and may be a glucose measurement device in which those members are integrally formed, and the insulin supply device is not limited to an insulin supply device in which an analysis element and a display section are connected to each other through a wiring, and may be an insulin supply device in which those members are integrally formed.

### Examples

Next, specific examples of the invention will be described.

### 1. Study of Examples

### 1-1. Production of Detection Section

### Example 1

<1> First, a transparent glass substrate having an average thickness of 0.5 mm was prepared as a substrate 301.
   The substrate 301 in which when the lengths of a first region 351, a second region 352, a third region 353, and a fourth region 354 were denoted by L1, L2, L3, and L4, respectively, and the widths of the first region 351 and the third region 353 were denoted by W1 and W2, respectively, the relationship of the magnitudes thereof satisfies the relationship shown in Table 1 was prepared.
<2> Subsequently, on the glass substrate, a patterned platinum film having an average thickness of 200 nm was formed by a mask sputtering method as an electrically conductive layer (electrode layer) 315 including a working electrode 311, a counter electrode 312, and a reference electrode 313.
   The working electrode 311, the counter electrode 312, and the reference electrode 313 were formed in the first region 351 at positions that satisfy the first configuration shown in FIG. 7, and the long sides and the short sides of the respective electrodes were made to satisfy the magnitudes shown in Table 1.
<3> Subsequently, 5 mg of GOD (glucose oxidase, activity: 150 U/mg) was dissolved in 6.1 g of ultrapure water, whereby a GOD solution was prepared. Then, 1.22 g of the obtained GOD solution was weighed and mixed with 1.0 g of PVA-SbQ (a 13.3% aqueous solution), whereby an analyte sensing layer forming material was prepared.
   Then, the analyte sensing layer forming material was applied onto the entire surfaces of the working electrode, the counter electrode, and the reference electrode using a spin-coating method under the conditions of 1000 rpm for 300 seconds while dropping the material thereon with a syringe, whereby a liquid coating film with a film thickness of 0.8 µm was formed, followed by natural drying for 5 minutes, whereby a thin film was obtained. Thereafter, the film was irradiated with ultraviolet light at 365 nm for 5 minutes to form an insolubilized film (cured film), whereby an analyte sensing layer 321 was formed.
<4> Subsequently, 0.45 g of PPG (polypropylene glycol) triol type (300), 0.5 g of toluene, 0.5 g of isophorone diisocyanate, and one drop of diazabicycloundecene (DBU) were mixed while stirring, whereby an analyte control layer forming material was prepared.
   Then, the analyte control layer forming material was applied onto the entire surface of the analyte sensing layer using a spin-coating method under the conditions of 1000 rpm for 300 seconds while dropping the analyte control layer forming material thereon with a syringe, and then, left for 24 hours to form urethane, whereby an analyte control layer 322, which has a film thickness of 0.2 µm, has a cross-linked structure including a urethane bond, and is configured to have an isocyanate group remaining when forming the urethane bond, was formed.
   By undergoing the steps as described above, a detection section 300 of Example 1 having the configuration shown in FIG. 7 was produced.

### Example 2

A detection section 300 of Example 2 having the configuration shown in FIG. 7 was produced in the same manner as in the above-mentioned Example 1 except that as the substrate 301 prepared in the step <1>, a substrate having the lengths (L1 to L4) shown in Table 1 was used.

**Table 1**

| | Width | | Length | | | | Working electrode | | Counter electrode | | Reference electrode | |
|---|---|---|---|---|---|---|---|---|---|---|---|---|
| | W1 | W2 | L1 | L2 | L3 | L4 | Long side | Short side | Long side | Short side | Long side | Short side |
| Example 1 | 0.8 | 0.4 | 2.5 | 2.5 | 5.0 | 5.0 | 2.0 | 0.2 | 2.0 | 0.2 | 2.0 | 0.2 |
| Example 2 | 0.8 | 0.4 | 2.5 | 7.5 | 0.0 | 5.0 | 2.0 | 0.2 | 2.0 | 0.2 | 2.0 | 0.2 |

| | | | | | | | | | | | | |
|---|---|---|---|---|---|---|---|---|---|---|---|---|
| Unit: mm | | | | | | | | | | | | |

### Examples 3 and 4

Detection sections 300 of Examples 3 and 4 having the configuration shown in FIG. 11 were produced in the same manner as in the above-mentioned Example 1 except that as the substrate 301 prepared in the step <1>, a substrate having the widths (W1 and W2) and the lengths (L1 to L4) shown in Table 2 was used, and further, in the step <2>, the working electrode 311, the counter electrode 312, and the reference electrode 313 were formed in the first region 351 at positions that satisfy the second configuration shown in FIG. 11, and the long sides and the short sides of the respective electrodes were made to satisfy the magnitudes shown in Table 2.

**Table 2**

| | Width | | Length | | | | Working electrode | | Counter electrode | | Reference electrode | |
|---|---|---|---|---|---|---|---|---|---|---|---|---|
| | W1 | W2 | L1 | L2 | L3 | L4 | Long side | Short side | Long side | Short side | Long side | Short side |
| Example 3 | 0.4 | 0.2 | 4.5 | 1.5 | 4.0 | 5.0 | 2.0 | 0.2 | 2.0 | 0.2 | 2.0 | 0.05 |
| Example 4 | 0.4 | 0.2 | 4.5 | 5.5 | 0 | 5.0 | 2.0 | 0.2 | 2.0 | 0.2 | 2.0 | 0.05 |

| | | | | | | | | | | | | |
|---|---|---|---|---|---|---|---|---|---|---|---|---|
| Unit: mm | | | | | | | | | | | | |

### Example 5

A detection section 300 of Example 5 having the configuration shown in FIG. 12 was produced in the same manner as in the above-mentioned Example 1 except that as the substrate 301 prepared in the step <1>, a substrate having the widths (W1 and W2) and the lengths (L1 to L4) shown in Table 3 was used, and further, in the step <2>, the working electrode 311, the counter electrode 312, and the reference electrode 313 were formed in the first region 351 at positions that satisfy the third configuration shown in FIG. 12, and the long sides and the short sides of the respective electrodes were made to satisfy the magnitudes shown in Table 3.

**Table 3**

| | Width | | Length | | | | Working electrode | | Counter electrode | | Reference electrode | |
|---|---|---|---|---|---|---|---|---|---|---|---|---|
| | W1 | W2 | L1 | L2 | L3 | L4 | Long side | Short side | Long side | Short side | Long side | Short side |
| Example 5 | 0.3 | 0.15 | 4.5 | 5.5 | 0 | 5.0 | 2.0 | 0.2 | 2.0 | 0.15 | 2.0 | 0.05 |

| | | | | | | | | | | | | |
|---|---|---|---|---|---|---|---|---|---|---|---|---|
| Unit: mm | | | | | | | | | | | | |

### Examples 6 and 7

Detection sections 300 of Examples 6 and 7 having the configuration shown in FIG. 13 were produced in the same manner as in the above-mentioned Example 1 except that as the substrate 301 prepared in the step <1>, a substrate having the widths (W1 and W2) and the lengths (L1 to L4) shown in Table 4 was used, and further, in the step <2>, the working electrode 311, the counter electrode 312, and the reference electrode 313 were formed in the first region 351 at positions that satisfy the fourth configuration shown in FIG. 13, and the long sides and the short sides of the respective electrodes were made to satisfy the magnitudes shown in Table 4.

**Table 4**

| | Width | | Length | | | | Working electrode | | Counter electrode | | Reference electrode | |
|---|---|---|---|---|---|---|---|---|---|---|---|---|
| | W1 | W2 | L1 | L2 | L3 | L4 | Long side | Short side | Long side | Short side | Long side | Short side |
| Example 6 | 0.4 | 0.2 | 6.5 | 1.5 | 4.0 | 5.0 | 2.0 | 0.2 | 2.0 | 0.2 | 2.0 | 0.2 |
| Example 7 | 0.3 | 0.15 | 6.5 | 1.5 | 4.0 | 5.0 | 2.0 | 0.15 | 2.0 | 0.15 | 2.0 | 0.15 |

| | | | | | | | | | | | | |
|---|---|---|---|---|---|---|---|---|---|---|---|---|
| Unit: mm | | | | | | | | | | | | |

### 1-2. Evaluation

With respect to a previously prepared needle section 121, each of the detection sections 300 of the respective Examples was inserted into a hollow section 114 so as to be in a state shown in FIG. 10.

As the needle section 121, needle sections in which the shape of the cross section is an ellipse or a circle (true circle) were separately prepared as those satisfying the relationship of the lengths as shown in Table 5, and each of the detection sections 300 of the respective Examples was inserted into each of the needle sections.

Then, the needle section 121 in which the detection section 300 was disposed in the hollow section 114 was stuck in a skin model, and thereafter, the needle section 121 was detached alone. That is, the needle section 121 was removed from the skin model in a state where the detection section 300 was placed in the skin model.

Thereafter, the occurrence of a break in the detection section 300 placed in the skin model was confirmed by visual observation.

The results are shown in Table 5.

**Table 5**

| | Detection section | | Needle section | | | | | Evaluation |
|---|---|---|---|---|---|---|---|---|
| | | | Relationship of length (shape) | | | | Shape of cross section | |
| | W1 | W2 | PT | P0 | P1-a | P1-b | circle/ellipse | presence or absence of break |
| Example 1A | 0.8 | 0.4 | 1.0 | 0.6 | 0.8 | 0.8 | circle | absent |
| Example 1B | 0.8 | 0.4 | 1.0 | 0.6 | 0.8 | 0.8 | ellipse | absent |
| Example 2A | 0.8 | 0.4 | 1.0 | 0.6 | 0.8 | 0.8 | circle | absent |
| Example 2B | 0.8 | 0.4 | 1.0 | 0.6 | 0.8 | 0.8 | ellipse | absent |
| Example 3A | 0.4 | 0.2 | 0.6 | 0.3 | 0.45 | 0.45 | circle | absent |
| Example 3B | 0.4 | 0.2 | 0.6 | 0.3 | 0.45 | 0.45 | ellipse | absent |
| Example 4A | 0.4 | 0.2 | 0.5 | 0.3 | 0.4 | 0.4 | circle | absent |
| Example 4B | 0.4 | 0.2 | 0.5 | 0.3 | 0.4 | 0.4 | ellipse | absent |
| Example 5A | 0.3 | 0.15 | 0.4 | 0.25 | 0.32 | 0.32 | circle | absent |
| Example 5B | 0.3 | 0.15 | 0.4 | 0.25 | 0.32 | 0.32 | ellipse | absent |
| Example 6A | 0.4 | 0.2 | 0.5 | 0.3 | 0.4 | 0.4 | circle | absent |
| Example 6B | 0.4 | 0.2 | 0.5 | 0.3 | 0.4 | 0.4 | ellipse | absent |
| Example 7A | 0.3 | 0.15 | 0.4 | 0.25 | 0.32 | 0.32 | circle | absent |
| Example 7B | 0.3 | 0.15 | 0.4 | 0.25 | 0.32 | 0.32 | ellipse | absent |

As shown in Table 5, in the case of the detection sections 300 of the respective Examples, the detection sections 300 could be inserted without causing a break in the detection sections 300 with respect to the hollow section 114 of the needle section 121.

### 2. Study of Comparative Examples

### 1-1. Production of Detection Section

### Comparative Example 1

A detection section 300 of Comparative Example 1 in which the configuration shown in FIG. 7 was adopted as the arrangement of the working electrode 311, the counter electrode 312, and the reference electrode 313 was produced in the same manner as in the above-mentioned Example 1 except that as the substrate 301 prepared in the step <1>, a substrate having the widths (W1 and W2) and the lengths (L0 and L4) shown in Table 6 was used.

In Comparative Example 1, W1 = W2, and the widths of the first region 351 to the fourth region 354 were the same, and therefore, the sum of the lengths L1, L2, and L3 of the first region 351, the second region 352, and the third region 353 (L1+L2+L3) is denoted by L0.

**Table 6**

| | Width | | Length | | Working electrode | | Counter electrode | | Reference electrode | |
|---|---|---|---|---|---|---|---|---|---|---|
| | W1 | W2 | L0 | L4 | Long side | Short side | Long side | Short side | Long side | Short side |
| Comparative Example 1 | 0.8 | 0.8 | 10 | 5.0 | 2.0 | 0.2 | 2.0 | 0.2 | 2.0 | 0.2 |

| | | | | | | | | | | |
|---|---|---|---|---|---|---|---|---|---|---|
| Unit: mm | | | | | | | | | | |

### Comparative Example 2

A detection section 300 of Comparative Example 2 in which the configuration shown in FIG. 11 was adopted as the arrangement of the counter electrode 312 and the reference electrode 313 was produced in the same manner as in the above-mentioned Example 1 except that as the substrate 301 prepared in the step <1>, a substrate having the widths (W1 and W2) and the lengths (L0 and L4) shown in Table 7 was used, and further, in the step <2>, the working electrode 311, the counter electrode 312, and the reference electrode 313 were formed in the first region 351 at positions that satisfy the second configuration shown in FIG. 11, and the long sides and the short sides of the respective electrodes were made to satisfy the magnitudes shown in Table 7.

In Comparative Example 2, W1 = W2, and the widths of the first region 351 to the fourth region 354 were the same, and therefore, the sum of the lengths L1, L2, and L3 of the first region 351, the second region 352, and the third region 353 (L1+L2+L3) is denoted by L0.

**Table 7**

| | Width | | Length | | Working electrode | | Counter electrode | | Reference electrode | |
|---|---|---|---|---|---|---|---|---|---|---|
| | W1 | W2 | L0 | L4 | Long side | Short side | Long side | Short side | Long side | Short side |
| Comparative Example 2 | 0.4 | 0.4 | 10 | 5.0 | 2.0 | 0.2 | 2.0 | 0.2 | 2.0 | 0.2 |

| | | | | | | | | | | |
|---|---|---|---|---|---|---|---|---|---|---|
| Unit: mm | | | | | | | | | | |

### Comparative Example 3

A detection section 300 of Comparative Example 3 in which the configuration shown in FIG. 12 was adopted as the arrangement of the counter electrode 312 and the reference electrode 313 was produced in the same manner as in the above-mentioned Example 1 except that as the substrate 301 prepared in the step <1>, a substrate having the widths (W1 and W2) and the lengths (L0 and L4) shown in Table 8 was used, and further, in the step <2>, the working electrode 311, the counter electrode 312, and the reference electrode 313 were formed in the first region 351 at positions that satisfy the third configuration shown in FIG. 12, and the long sides and the short sides of the respective electrodes were made to satisfy the magnitudes shown in Table 8.

In Comparative Example 3, W1 = W2, and the widths of the first region 351 to the fourth region 354 were the same, and therefore, the sum of the lengths L1, L2, and L3 of the first region 351, the second region 352, and the third region 353 (L1+L2+L3) is denoted by L0.

**Table 8**

| | Width | | Length | | Working electrode | | Counter electrode | | Reference electrode | |
|---|---|---|---|---|---|---|---|---|---|---|
| | W1 | W2 | L0 | L4 | Long side | Short side | Long side | Short side | Long side | Short side |
| Comparative Example 3 | 0.3 | 0.3 | 10 | 5.0 | 2.0 | 0.2 | 2.0 | 0.2 | 2.0 | 0.2 |

| | | | | | | | | | | |
|---|---|---|---|---|---|---|---|---|---|---|
| Unit: mm | | | | | | | | | | |

### 2-2. Evaluation

With respect to a previously prepared needle section, each of the detection sections 300 of the respective Comparative Examples was disposed in a concave portion included in the needle section.

As the needle section 121, needle sections in which the shape of the cross section is a semicircle and a concave portion is included were separately prepared as those satisfying the relationship of the lengths as shown in Table 9, and each of the detection sections 300 of the respective Comparative Examples was disposed in the concave portion of each of the needle sections. In Table 9, "PT" denotes the width of the opening section (slit), and "P2" denotes the maximum depth of the concave portion (opening section).

Then, the needle section in which the detection section 300 was disposed in the concave portion was stuck in a skin model, and thereafter, the needle section was detached alone. That is, the needle section was removed from the skin model in a state where the detection section 300 was placed in the skin model.

Thereafter, the occurrence of a break in the detection section 300 placed in the skin model was confirmed by visual observation.

The results are shown in Table 9.

**Table 9**

| | Detection section | | Needle section | | | Result of insertion |
|---|---|---|---|---|---|---|
| | | | Relationship of length (shape) | | Shape of cross section | |
| | W1 | W2 | PT | P2 | | presence or absence of break |
| Comparative Example 1 | 0.8 | 0.8 | 1.0 | 0.5 | semicircle | A break occurred in the detection section at the time of insertion with a probability of 50% or more. |
| Comparative Example 2 | 0.4 | 0.4 | 0.7 | 0.4 | semicircle | A break occurred in the detection section at the time of insertion with a probability of 70% or more. |
| Comparative Example 3 | 0.3 | 0.3 | 0.6 | 0.3 | semicircle | A break occurred in the detection section at the time of insertion with a probability of 80% or more. |

As shown in Table 9, in the case of the detection sections 300 of the respective Comparative Examples, the detection section 300 could not be maintained in the needle section when the detection section 300 was inserted (stuck) into the skin model, resulting in the occurrence of a break in the detection section 300 due to this.

## Claims

1. A sensor substrate, which is adapted to be inserted into a living body by being guided by an insertion needle to be stuck and inserted into the living body, comprising:
a first region which is provided in a tip end portion of the sensor substrate and includes a detection electrode;
a third region which includes a wiring section and has a smaller width than the width of a slit of the insertion needle; and
a second region which is provided between the first region and the third region and has a width gradually decreasing from the width of the first region to the width of the third region.

2. The sensor substrate according to claim 1, wherein the first region and the third region are each formed with a constant width and preferably each have a rectangular shape.

3. The sensor substrate according to claim 1 or 2 wherein the width of the first region is larger than the width of the third region.

4. The sensor substrate according to claim 3, wherein the ratio of the width of the third region to the width of the first region is larger than 1/2 and smaller than 4/5.

5. The sensor substrate according to any one of the preceding claims, wherein the angle of the outer surfaces of the first region and the second region is 120° or more.

6. The sensor substrate according to any one of the preceding claims, wherein the sensor substrate includes two electrodes disposed in a parallel direction intersecting the long axis direction, and an electrode disposed in a series direction along the long axis direction of at least one of the electrodes in the first region.

7. An analysis element, comprising the sensor substrate according to any one of the preceding claims and an insertion needle having a hollow section in which the sensor substrate is disposed.

8. The analysis element according to claim 7, wherein the hollow section comprises a slit having a smaller width than the first region of the sensor substrate, and the width of the first region is preferably smaller than the width of the hollow section.

9. The analysis element according to claim 8, wherein the width of the third region of the sensor substrate is smaller than the width of the slit of the hollow section.

10. A glucose measurement device, comprising the analysis element according to any one of claims 6 to 9.

11. An insulin supply device, comprising the glucose measurement device according to claim 10.
